# EUROPEAN PATENT SPECIFICATION

(11) **EP 1 849 489 B1**
(45) Date of publication and mention of the grant of the patent: **07.10.2009**
(21) Application number: 07015497.6
(22) Date of filing: 01.05.1998
(51) Int. Cl.: A61M 5/00, A61B 17/34, A61M 39/06

(54) **Cannula assembly**
Kanülenanordnung
Ensemble de canule

(30) Priority: 02.05.1997 US 45412 P
(43) Date of publication of application: 31.10.2007
(62) Divisional of application: 05024301.3
(73) Proprietor: United States Surgical Corporation, Norwalk, Connecticut 06856 (US)
(72) Inventor: Racenet, David C., Southbury,CT 06488 (US); Stellon, Gene A., Southington,CT 06489 (US); Vumback, William J., Northford ,CT 06472 (US); Pasqualucci, Joseph, Southbury,CT 06488 (US)
(74) Representative: HOFFMANN EITLE

(56) References cited:
- DE-A1- 3 737 121
- US-A- 4 447 237
- US-A- 5 391 153
- US-A- 5 391 154
- US-A- 5 463 010
- US-A- 5 603 702

## Description

### BACKGROUND

### 1. Technical Field

The present disclosure relates to seal systems of the type adapted to allow the introduction of a surgical instrument into a patient's body. In particular, the disclosure relates to a seal system to be used in combination with a cannula assembly where the cannula assembly is intended for insertion into a patient's body and an instrument is inserted into the patient's body through the cannula.

### 2. Background Of Related Art

Laparoscopic procedures are performed in the interior of the abdomen through a small incision, e.g., through narrow endoscopic tubes or cannulas inserted through a small entrance incision in the skin. Minimally invasive procedures are performed elsewhere in the body, e.g., in the chest, and are often generally referred to as "endoscopic" procedures. Minimally invasive or endoscopic procedures generally require that any instrumentation inserted into the body be sealed, i.e. provisions must be made to ensure that gases do not enter or exit the body through the endoscopic incision as, for example, in surgical procedures in which the surgical region is insufflated. Moreover, endoscopic procedures often require the surgeon to act on organs, tissues, and vessels far removed from the incision, thereby requiring that any instruments used in such procedures be relatively long and narrow.

For such procedures, the introduction of a tube into certain anatomical cavities such as the abdominal cavity is usually accomplished by use of a system incorporating a trocar and cannula assembly. A cannula assembly is formed of a cannula attached to a cannula housing which generally includes seal assembly adapted to maintain a seal across the opening of the seal assembly both with and without an instrument inserted therethrough. Since the cannula is in direct communication with the internal portion of the seal assembly, insertion of the cannula into an opening in the patient's body so as to reach the inner abdominal cavity should be adapted to maintain a fluid tight interface between the abdominal cavity and the outside atmosphere.

Since minimally invasive surgical procedures in the abdominal cavity of the body generally require insufflating gases to raise the cavity wall away from vital organs, the procedure is usually initiated by use of a Verres needle through which a gas is introduced into the body cavity. The gas provides a slight pressure which raises the wall surface of the peritoneum away from the vital organs thereby providing an adequate region in which to operate. Thereafter, a trocar assembly which includes a cannula and a trocar or obturator is inserted within the cannula to puncture the peritoneum, i.e. the inner lining of the abdominal cavity wall. The obturator is removed and laparoscopic or endoscopic surgical instruments may then be inserted through the cannula to perform surgery within the abdominal cavity. The cannula may also be-utilized for introducing tubes into the body as for drainage purposes, for specimen removal, for diagnostic evaluations, or the like.

In view of the need to maintain the atmospheric integrity of the inner area of the cavity, a seal assembly for a cannula which permits introduction of an obturator and a wide range of surgical instruments and which maintains the atmospheric integrity of the inner area of the cavity is desirable.. Generally, in the context of insufflatory, minimally invasive surgical procedures, cannula assemblies include structure(s) that satisfy two sealing requirements. The first requirement is to provide a substantially fluid tight seal when an instrument is not present in the cannula. The second requirement is to provide a substantially fluid tight seal when an instrument is being introduced into or already is present in the cannula. In this regard, there have been a number of attempts in the prior art to provide such sealing requirements.

U.S. Patent No. 4,655,752 to Honkanen et al. teaches a cannula including a housing and first and second seal members. The first seal member is conically tapered toward the bottom of the housing and has a circular opening in its center, while the second seal is conically tapered and cup shaped. The second seal includes at least one slit to allow for the passage of instruments.

U.S. Patent No. 4,929,235 to Merry et al. teaches a self-sealing catheter introducer having a sealing mechanism to prevent blood or fluid leakage. The sealing mechanism includes a planar sealing element having a slit and a conical sealing element. The sealing elements are each adapted to surround a tube.

U.S. Patent Nos'. 4,874,377 and 5,064,416 to Newgard et al. relate to a self-occluding intravascular cannula assembly in which an elastomeric valving member is positioned transversely to a housing and is peripherally compressed to cause displacement, distortion and/or rheological flow of the elastomeric material. A frustoconical dilator projection cooperates with the elastomeric valving member in moving the valving member to a non-occluding position.

U.S. Patent No. 5,300,033 to Miller suggests a valve construction including an elastic body having a cylindrical wall with first and second walls formed integrally with the cylindrical wall. The second wall includes a slit to permit passage of a surgical instrument and first and second leaflets which define the slit. The leaflets are thicker in cross section to provide an additional closing force at the slit.

A disadvantage of several known seal systems for cannulas concerns the difficulty encountered in inserting and advancing the surgical instrument through the seal unit. In particular, since known elastomeric seal members are designed to form and maintain a fluid tight seal about the instrument, the aperture or slit within the seal through which the instrument is passed is of relatively small or narrow dimension. Further, portions of the seal member defining the aperture are generally thick in cross-section to provide a sufficient closing force of the seal about the instrument. see, e.g., U.S. Patent No. 5,300,033. As a consequence of these design considerations, the level of force needed to insert and advance the instrument through the seal aperture is increased, thereby requiring awkward maneuvering on the surgeon's behalf to appropriately position the instrument for the desired surgery. Moreover, known seal systems are generally ineffectual in accommodating instruments of differing diameter while maintaining acceptable insertion forces and facilitating the range of desired surgical manipulations, e.g., angular instrument movements and specimen removal.

Accordingly, the present disclosure obviates the disadvantages of the prior art by providing a seal unit or assembly for a cannula assembly, which is capable of forming and maintaining a tight seal about instruments of varying diameters inserted through the cannula and which incorporates structure to enhance and facilitate passage of the instrument through the seal unit.

### SUMMARY

The present invention discloses a cannula assembly as defined in claim 1. The present disclosure provides, but this is not recited by claim 1, a seal assembly for reception of an elongated surgical instrument, which comprises a body having at least one opening configured and dimensioned to permit entry of an elongated surgical instrument and defining a central longitudinal axis; a seal member formed of a resilient material and defining an aperture therein, the aperture being configured and dimensioned such that insertion of the surgical instrument into the aperture causes the resilient material defining the aperture to resiliently contact the outer surface of the surgical instrument in a substantially fluid tight manner; and a fabric layer juxtaposed relative to the resilient material.

The seal assembly may further include a coating applied to the seal member to reduce friction between the seal member and surgical instrumentation inserted therein. The coating is preferably a hydrocyclosiloxane membrane prepared by plasma polymerization process.

In one aspect of the presently disclosed seal assembly a ring member is secured to the seal member and includes a dampening element disposed between a surface of the ring member and a surface of the body.

### BRIEF DESCRIPTION OF THE DRAWINGS

Various embodiments are described herein below with reference to the drawings wherein:
FIG. 1 is a perspective view of one embodiment of a seal assembly constructed in accordance with the present disclosure;
FIG. 2 is a perspective view with parts separated showing the various structural components of the seal assembly embodiment of FIG. 1;
FIG. 3 is a perspective view of a fabric portion for incorporation into the seal element of the embodiment of FIG. 1;
FIG. 4 is a cross-sectional view taken along section line 4-4 of FIG. 3;
FIG. 5 is a perspective view of a fully formed seal member for the seal assembly of FIG. 1;
FIG. 6 is a cross-sectional view taken along section line 6-6 of FIG. 5;
FIG. 6A is an alternative embodiment of the seal element of FIG. 6;
FIG. 7 is a cross-sectional view of the seal assembly of FIG. 1;
FIG. 8 is a perspective view of a trocar assembly having the seal assembly of FIG. 1 removably installed thereon;
FIG. 9 is a partial cross-sectional view showing the seal body housing taken along section line 9-9 of FIG. 8;
FIG. 10 is an alternative embodiment of the seal assembly of the present disclosure;
FIG. 11 is a cross-sectional view of a further embodiment of the seal assembly constructed in accordance with the present disclosure;
FIG. 12 is a perspective view of the seal assembly of FIG. 11;
FIG. 13 is a perspective view with parts separated of the seal assembly embodiment of FIG. 11;
FIG. 14 is a cross-sectional view of a further embodiment of.the seal assembly constructed in accordance with the present disclosure; and
FIG. 15 is a perspective view of the seal member of the seal assembly of FIG. 14.

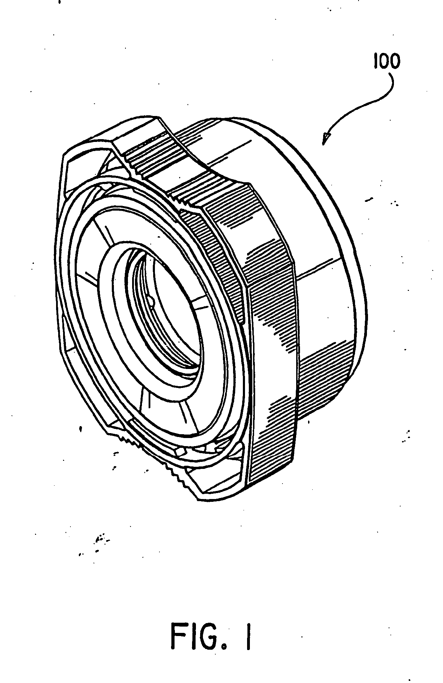

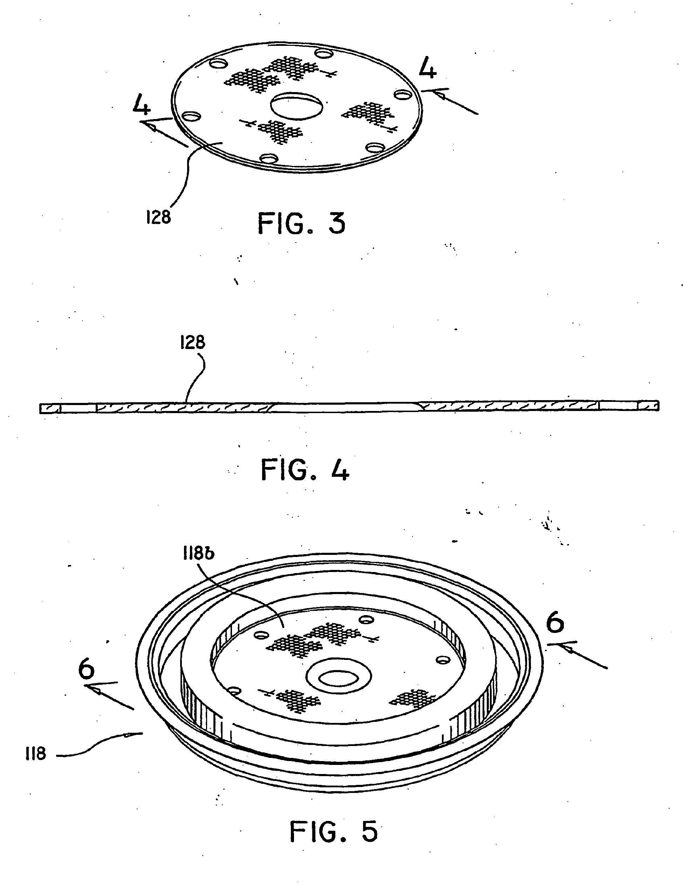

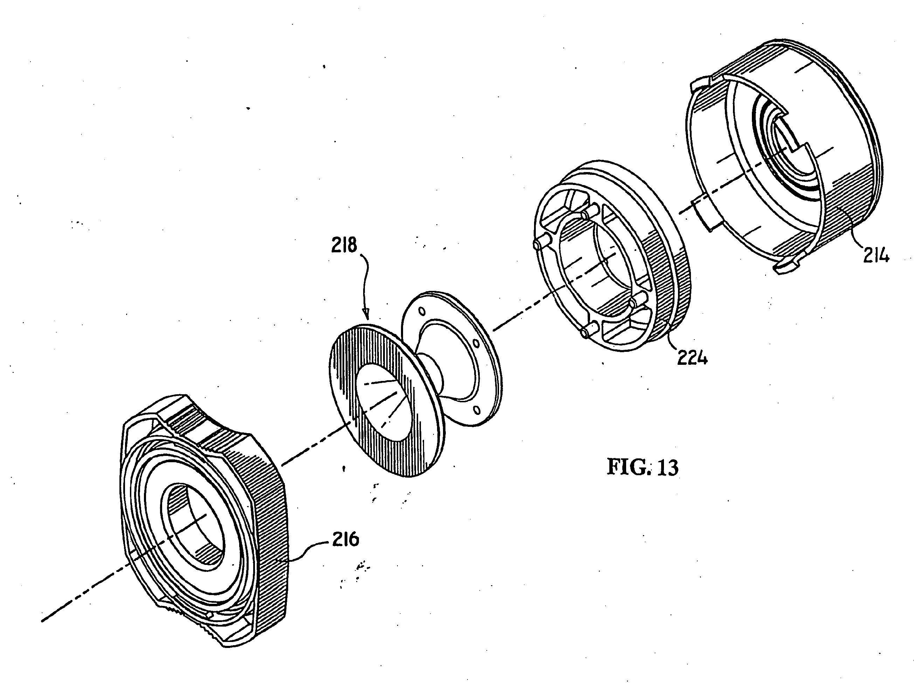

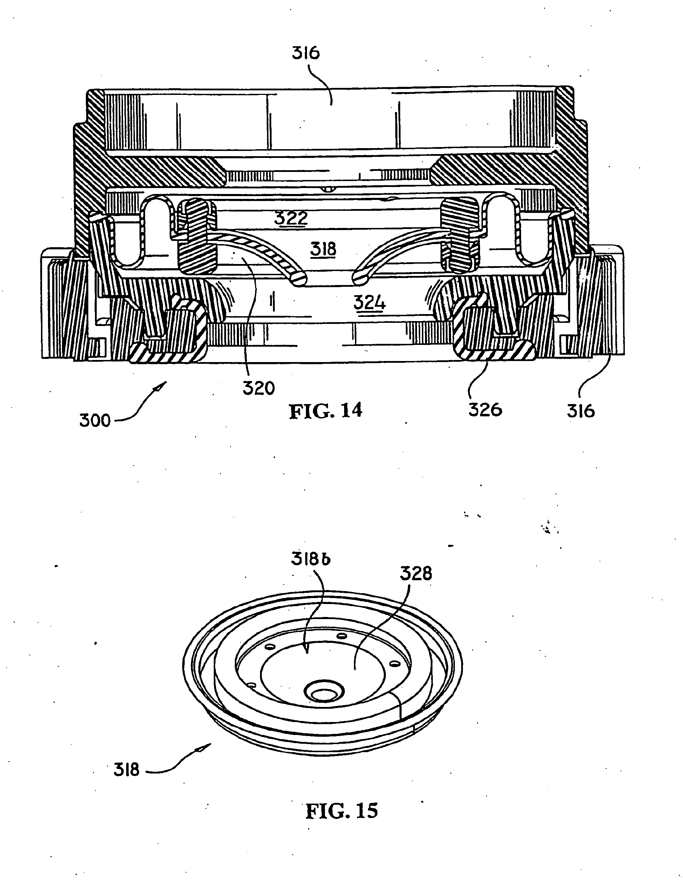

Claim 1 is concerned with a sealing member having a conical shape. Figures 14 and 15 and the associated description disclose an embodiment of such a sealing member. Figures 1 to 13 and the associated description do not disclose a sealing member so shaped, but provide information useful for a full understanding of the invention.

### DETAILED DESCRIPTION OF PREFERRED EMBODIMENTS

Referring now in detail to the drawing figures in which like reference numerals identify similar or identical elements, a first embodiment of the seal assembly of the present disclosure is illustrated in FIGs. 1-9, and is designated generally as seal assembly 100.

The presently disclosed seal assembly embodiments contemplate the introduction of various types of surgical instruments adapted for insertion through an elongated trocar assembly. Examples of such instruments include clip appliers, graspers, dissectors, retractors, staplers, laser fibers, photographic devices, endoscopes and laparoscopes, tubes, and the like. Such instruments are collectively referred to herein as "instruments".

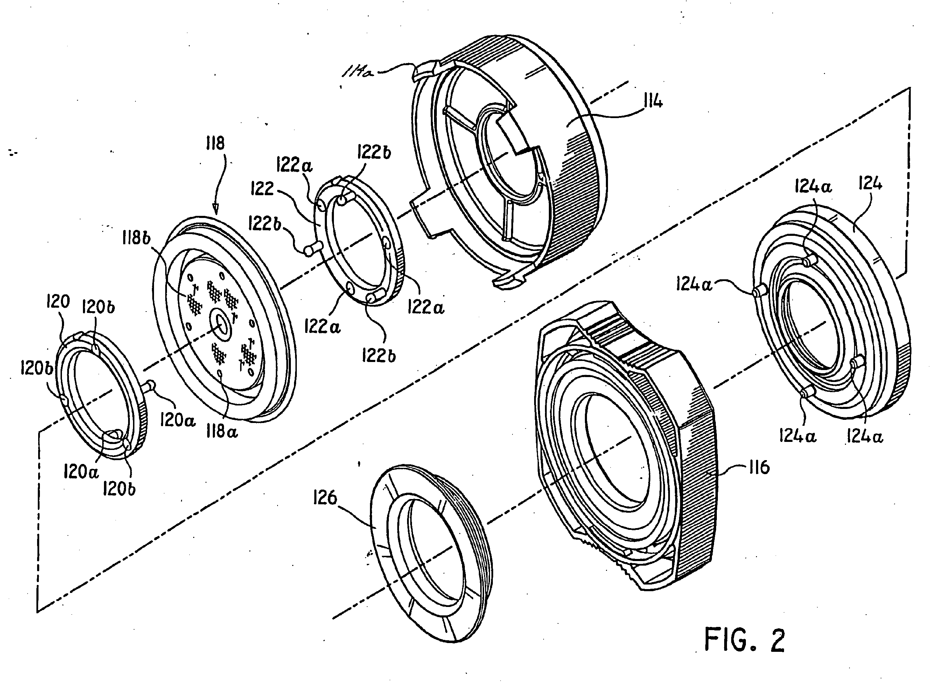

Referring to FIG. 2, seal assembly 100 includes a seal member 118 disposed within the seal assembly body or housing components 114 and 116 which snap fit together by way of flexible tab portions 114a being deflected upon insertion into receiving openings (not shown) formed in housing component 116. Seal member 118 has a circular array of holes formed therethrough around the periphery of an inner section 118b.

A two part ring assembly which includes ring members 120 and 122 are snap fitted together on either side of seal member 118. Ring member 120 is disposed adjacent the distally facing surface of seal member 118 and ring member 122 is disposed on the proximally facing side of seal member 118. Ring 120 is provided with holes 120a and posts 120b which are alternately disposed around the ring and are aligned with holes 118a on seal member 118. Ring 122 is provided with posts 122a and holes 122b which mate with holes 120a and posts 120b of ring member 120, respectively by snap fitting together thereby surrounding inner section 118b. Although rings 120 and 122 are shown having alternating holes and posts, one of the rings could have all holes formed therein while the other ring could have all posts aligned with the holes of the other ring. Additionally, greater or fewer holes and posts may be utilized to secure the two rings together.

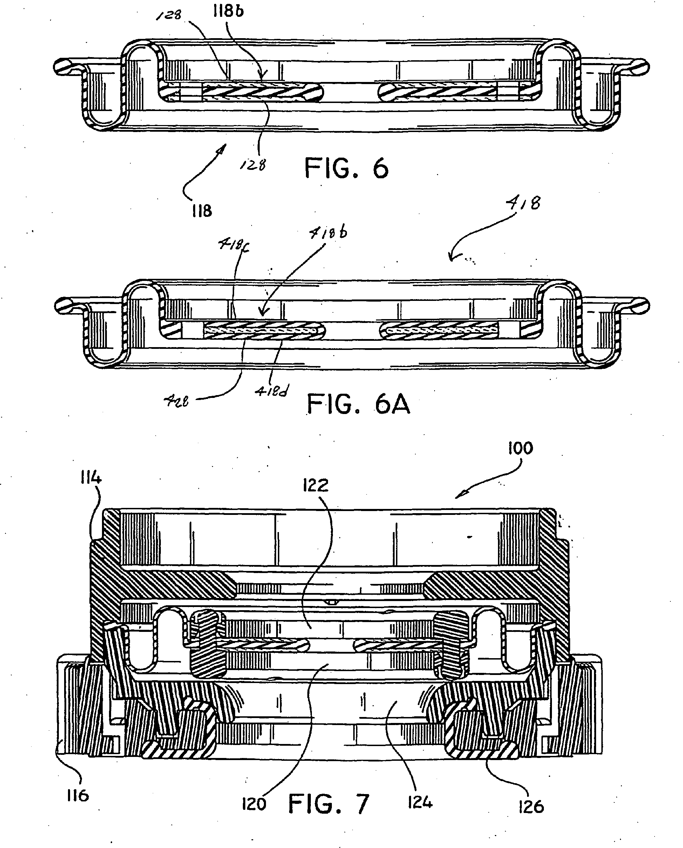

A seal clamp 124 is provided within the housing component 114 and 116 and serves to secure the outer periphery of seal member 118 within seal assembly 100 (as best shown in FIG. 7). Seal clamp 124 is provided with four projecting posts 124a which fit within openings (FIG. 7) formed on the proximal side of lower housing 116. Seal clamp 124 also serves to secure a proximal flange of a lower seal 126 which is provided at the distal end of lower housing member 116. Lower seal 126 assists in the securement of seal assembly 100 to cannula assembly 110.

Referring now to FIGs . 3-6, seal member 118 includes a fabric disc-shaped portion 128 which is preferably disposed on both the proximal and distal sides of inner section 118b of seal member 118. Alternatively, but this alternative is not included by claim 1, fabric section 128 may be disposed on just one of either the proximally facing surface or the distally facing surface of inner portion 118b, as desired. Fabric portion 128 may be of any suitable fabric, for example, a SPANDEX material containing 20% LYCRA available from Milliken.

In one method of forming the composite seal member 118 with fabric portion 128 a raw, i.e., uncured polyisoprene plug is first compressed into a flat state, e.g., a flat sheet of polyisoprene. A single layer of fabric is positioned on top of the flattened polyisoprene sheet and compressed into the uncured rubber by any suitable compression process such as, for example, calendering. If it is desired to have fabric on both sides of seal member 118, this process is repeated on the other side of the polyisoprene sheet. The fabric polyisoprene composite is die cut into circular slugs having an outer diameter and an inner diameter which forms a central aperture. The slugs are placed in a hot compression mold to cure the polyisoprene. This step also serves to extrude the outer portions of seal member 118 which extend outwardly from inner section 118b.

During the above-described process the bleed-through of the polyisoprene material into and/or through the fabric layers is regulated by the density of the fabric selected. A greater degree of bleed-through of polyisoprene provides greater resistance to fraying of the fabric upon repeated insertion of instruments through the seal. However, too much bleed-through of the polyisoprene through the fabric will increase friction forces upon instruments being inserted through seal member 118.

Referring to FIG. 6A, an alternative embodiment of seal member 118 is shown as seal member 418. Seal member 418 is the same as seal member 118 in most aspects except that inner section 418b is formed to have fabric layer 428 enveloped between upper and lower polyisoprene layers 418c and 418d.

In order to reduce friction between instruments and the seal member, e.g. seal member 118 or seal member 418, as instruments are inserted through seal assembly 100, a coating may be applied to the seal member. One coating which has been found particularly effective is a hydrocyclosiloxane membrane prepared by plasma polymerization process. Such a coating is available from Innerdyne, Inc. of Salt Lake City, Utah, U.S.A., and is disclosed in U.S. Patent No. 5,463,010 which issued to Hu et al. on October 31, 1995.

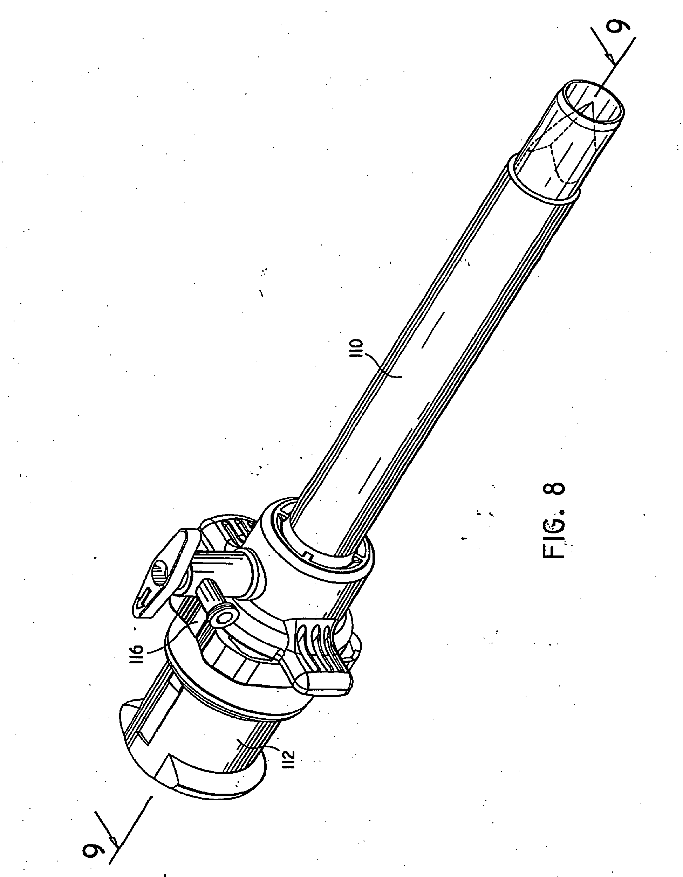

Referring to FIGs. 7 and 8, seal assembly 100 is used in combination with a conventional trocar assembly which includes a cannula assembly 110 and a trocar obturator 112. Examples of trocar assemblies in which the present seal assembly may be utilized are disclosed in U.S. Patent No. 5,603,702 which issued on February 18, 1997 to Smith et al. and U.S. Application Serial No. 08/546,009 filed October 20, 1995 by Smith et al.

Seal assembly 100, either alone or in combination with a seal unit/seal assembly internal to cannula assembly 110, provides a substantial seal between a body cavity of a patient and the outside atmosphere both during and subsequent to insertion of an instrument through the cannula. In this manner, insufflation gases are prevented from escaping through the trocar assembly to the outside environment. Seal assembly 100 is capable of accommodating instruments of varying diameter, e.g., from about 5 mm to about 12 mm, while providing a fluid tight seal with the outer diameter of each instrument. The versatility of the presently disclosed seal assembly embodiments greatly facilitate endoscopic surgery, wherein a variety of instruments having different diameters are often needed during a single surgical procedure.

Seal assembly 100 is preferably detachably mountable to the proximal end of cannula assembly 110. Thus, the surgeon can remove the seal assembly 100 from the cannula assembly 110 at any time during the surgical procedure and, similarly, mount the seal assembly 100 to the cannula when desired in order to provide a sealing engagement with an instrument to be inserted through the cannula. In addition, seal assembly 100 may be readily adapted for mounting to conventional cannulas of differing structures. The detachability of seal assembly 100 from cannula assembly 110 facilitates specimen removal through cannula assembly 110. Seal assembly 100 includes a housing which is formed by the snap fitting together of end cap 114 and lower housing member 116. Preferably the housing components of seal assembly 100 are formed of a polycarbonate material such as ABS available from the General Electric Company.

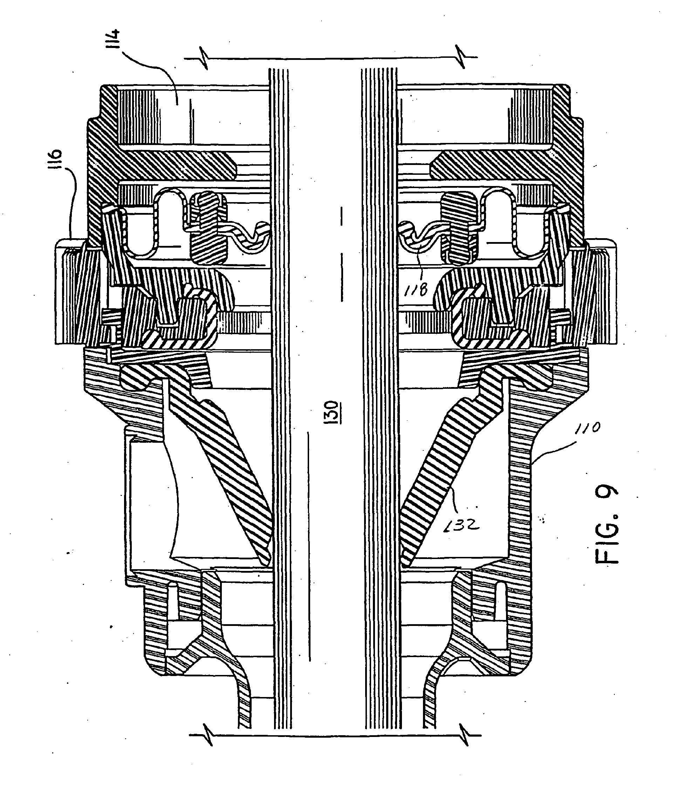

FIG. 9 shows an instrument having a shaft 130 inserted through seal assembly 100 and a duck bill valve or "zero" seal valve 132 which prevents the escape of insufflation gases in the absence of an instrument in the trocar assembly. As shown in FIG. 9, seal member 118 provides a seal about the periphery of shaft 130.

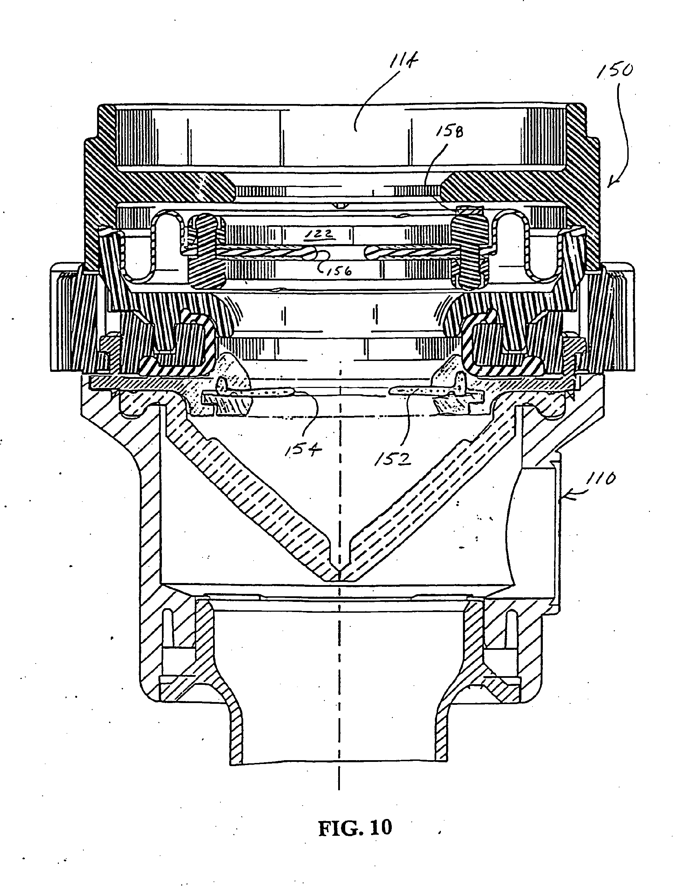

Referring to FIG. 10, an alternate embodiment of seal assembly 100 is designated generally as seal assembly 150. Seal assembly 150 is the same as seal assembly 100 except that an inner planar seal member 152 is disposed in the distal end of seal assembly 100 to provide additional sealing capability for instruments having larger diameters. Seal element 152 has an aperture 154 which has a diameter larger than the diameter of aperture 156 of seal member 118. A further feature illustrated in FIG. 10 is a dampening member such as pad 158 which is secured to the proximal surface of ring 122 to dampen the sound created by the impact of the proximal surface of ring 122 with the inner distal facing surface of housing component 114. Other dampening member configurations are also contemplated. For example, ring 122 may be over-molded with material such as polyisoprene so as to envelope part or all of the ring thereby forming a bumper between the ring and the housing component.

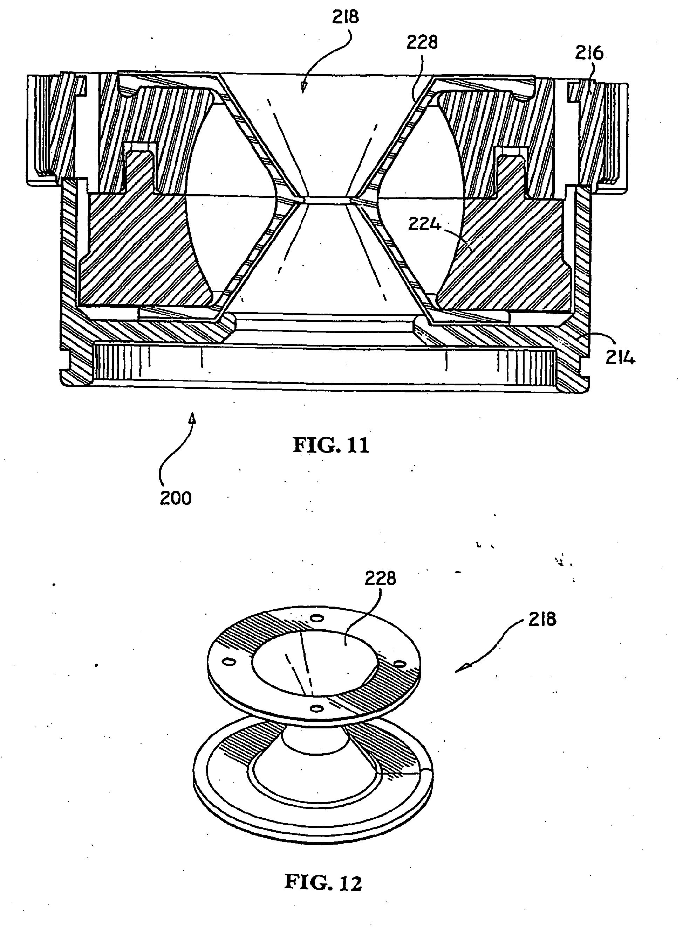

Referring to FIGs. 11-13, a further embodiment of a seal assembly generally designated as seal assembly 200 is shown throughout the several views. Seal member 218 is configured in an hourglass shape and preferably includes the fabric portion 228 formed as part of seal member 218 in a similar manner as described above. The friction reducing coating of a hydrocyclosiloxane membrane prepared by plasma polymerization process noted above may also be utilized to coat the surfaces of seal member 218.

Referring now to FIGs. 14 and 15, a further embodiment of the seal assembly generally designated as seal assembly 300 is shown. Seal member 318 is similar to seal member 118 except that inner portion 318b is formed in a conical shape with a wider opening directed towards a proximal end of seal assembly 300 and a narrower opening directed towards distal end of seal assembly 300. The friction reducing coating of a hydrocyclosiloxane membrane prepared by plasma polymerization process noted above may also be utilized to coat the surfaces of seal member 318.

## Claims

1. A cannula assembly (110) for use in a surgical procedure, comprising
a cannula member defining a longitudinal axis, the cannula member defining a longitudinal opening therethrough for passage of a surgical instrument; and
a sealing member (318) extending across the longitudinal axis and having a predetermined shape prior to insertion of the instrument, the sealing member including a resilient material, defining an opening for the receipt of the instrument and adapted to expand from an initial open condition to an expanded condition upon insertion of the instrument, the sealing member arranged so that insertion of the instrument causes the sealing member to resiliently contact the outer surface of the instrument to form a substantial seal therewith; **characterised by**
the sealing member having a conical shape, and a fabric portion (128) disposed on proximal and distal sides of the sealing member.

2. The assembly of claim 1, wherein the fabric portion comprises a layer of fabric.

3. The assembly of claim 1 or 2, wherein the fabric portion is compressed into the resilient material.

4. The assembly of claim 3, wherein the resilient material is at least bled-into the fabric.

5. The assembly of any one of the preceding claims, wherein the sealing member has a friction-reducing coating.

6. The assembly of claim 5, wherein the friction-reducing coating comprises a hydrocyclosiloxane membrane.

7. The assembly of any one of the preceding claims, wherein the sealing member has a proximal end and a distal end, the opening at the proximal end being wider than the opening at the distal end.

8. The assembly of any one of the preceding claims, further comprising a ring member (126) secured to the seal member.

9. The assembly of claim 8, wherein the ring member is disposed adjacent the distally facing side of the sealing member, another ring member (122) is disposed adjacent the proximally facing side of the sealing member, the ring members being snap fitted together.

10. The assembly of any one of the preceding claims, further comprising a housing (214, 216) and wherein the sealing member is disposed in the housing.

11. The assembly of claim 10, further comprising a zero closure seal (132) in the housing for preventing the escape of insufflation gases in the absence of an instrument.

12. The assembly of claim 9 or claim 10 or 11 when dependent on claim 9, wherein one or both of the rings is provided with posts (120b, 122a) mating with holes (120a, 122b) of the other ring and passing through holes (118a) formed through the fabric portions and the resilient material.

## Patentansprüche

1. Kanülenanordnung (110) zur Verwendung in einem chirurgischen Verfahren, umfassend
ein Kanülenelement, das eine Längsachse definiert, wobei das Kanülenelement eine Längsöffnung **dadurch** für einen Durchgang eines chirurgischen Instruments definiert; und
ein Abdichtelement (318), das sich über die Längsachse erstreckt und eine vorbestimmte Form vor einem Einsetzen des Instruments aufweist, wobei das Abdichtelement ein elastisches Material enthält, das eine Öffnung für die Aufnahme des Instruments definiert und angepasst ist, um sich von einem anfänglichen geöffneten Zustand zu einem expandierten Zustand nach einem Einsetzen des Instruments zu dehnen, wobei das Abdichtelement so angeordnet ist, dass ein Einsetzen des Instruments das Abdichtelement dazu bringt, die äußere Oberfläche des Instruments elastisch zu kontaktieren, um eine stoffliche Dichtung damit zu bilden, **gekennzeichnet durch**
das Abdichtelement mit einer konischen Form und einen Faserabschnitt (128) an proximalen und distalen Seiten des Abdichtelements.

2. Anordnung nach Anspruch 1, wobei der Faserabschnitt eine Lage Fasern umfasst.

3. Anordnung nach Anspruch 1 oder 2, wobei der Faserabschnitt in das elastische Material eingedrückt ist.

4. Anordnung nach Anspruch 3, wobei das elastische Material zumindest in die Faser gelaufen ist.

5. Anordnung nach einem der vorstehenden Ansprüche, wobei das Abdichtelement eine reibungsreduzierende Beschichtung aufweist.

6. Anordnung nach Anspruch 5, wobei die reibungsreduzierende Beschichtung eine Hydrozyklosiloxanmembran umfasst.

7. Anordnung nach einem der vorstehenden Ansprüche, wobei das Abdichtelement ein proximales Ende und ein distales Ende aufweist, wobei die Öffnung an dem proximalen Ende breiter als die Öffnung an dem distalen Ende ist.

8. Anordnung nach einem der vorstehenden Ansprüche, ferner umfassend ein Ringelement (126), das an dem Abdichtelement befestigt ist.

9. Anordnung nach Anspruch 8, wobei das Ringelement benachbart zu der in distale Richtung weisenden Seite des Abdichtelements angeordnet ist, wobei ein anderes Ringelement (122) benachbart zu der in proximale Richtung weisenden Seite des Abdichtelements angeordnet ist, wobei die Ringelemente miteinander in Schnappverbindung stehen.

10. Anordnung nach einem der vorstehenden Ansprüche, ferner umfassend ein Gehäuse (214, 216) und wobei das Abdichtelement in dem Gehäuse angeordnet ist.

11. Anordnung nach Anspruch 10, ferner umfassend eine Nullschlussdichtung (132) in dem Gehäuse um das Ausweichen von Insufflationsgasen in der Abwesenheit eines Instruments zu verhindern.

12. Anordnung nach Anspruch 9 oder Anspruch 10 oder 11 in Abhängigkeit von Anspruch 9, wobei einer oder beide der Ringe mit Stäben (120b, 122a) versehen ist, die in Öffnungen (120a, 122b) des anderen Rings passen und durch Öffnungen (118a), die durch die Faserabschnitte und das elastische Material geformt sind, gelangen.

## Revendications

1. Ensemble de canule (110) pour utilisation dans une procédure chirurgicale, comprenant
un élément de canule définissant un axe longitudinal, l'élément de canule définissant une ouverture longitudinale à travers celui-ci pour le passage d'un instrument chirurgical; et
un élément de joint (318) s'étendant sur l'axe longitudinal et ayant une forme prédéterminée avant l'insertion de l'instrument, l'élément de joint incluant un matériau élastique, définissant une ouverture pour la réception de l'instrument et apte à s'expanser d'un état initial ouvert à un état expansé lors de l'insertion de l'instrument, l'élément de joint étant agencé de façon que l'insertion de l'instrument amène l'élément de joint à venir élastiquement en contact avec la surface externe de l'instrument pour former un joint substantiel avec celui-ci, **caractérisé par**
l'élément de joint ayant une forme conique, et une portion d'étoffe (128) disposée sur les côtés proximal et distal de l'élément de joint.

2. Ensemble selon la revendication 1, où la portion d'étoffe comprend une couche d'étoffe.

3. Ensemble selon la revendication 1 ou 2, où la portion d'étoffe est comprimée dans le matériau élastique.

4. Ensemble selon la revendication 3, où le matériau élastique est au moins infiltré dans l'étoffe.

5. Ensemble selon l'une quelconque des revendications précédentes, où l'élément de joint possède un revêtement réducteur de friction.

6. Ensemble selon la revendication 5, où le revêtement réducteur de friction comprend une membrane en hydrocyclosiloxane.

7. Ensemble selon l'une quelconque des revendications précédentes, où l'élément de joint possède une extrémité proximale et une extrémité distale, l'ouverture à l'extrémité proximale étant plus large que l'ouverture à l'extrémité distale.

8. Ensemble selon l'une quelconque des revendications précédentes, comprenant en outre un élément annulaire (126) fixé à l'élément de joint.

9. Ensemble selon la revendication 8, où l'élément annulaire est disposé d'une manière adjacente au côté orienté distalement de l'élément de joint, un autre élément annulaire (122) est disposé d'une manière adjacente au côté orienté proximalement de l'élément de joint, les éléments annulaires étant assemblés par encliquetage.

10. Ensemble selon l'une quelconque des revendications précédentes, comprenant en outre un boîtier (214, 216), et où l'élément de joint est disposé dans le boîtier.

11. Ensemble selon la revendication 10, comprenant en outre un joint de fermeture zéro (132) dans le boîtier pour empêcher l'échappement des gaz d'insufflation en l'absence d'un instrument.

12. Ensemble selon la revendication 9 ou la revendication 10 ou 11 dépendant de la revendication 9, où un ou les deux anneaux présentent des montants (120b, 122a) correspondant à des trous (120a, 122b) de l'autre anneau et passant à travers des trous (118a) ménagés à travers les portions d'étoffe et le matériau élastique.
